(19) Europäisches Patentamt
European Patent Office
Office européen des brevets

(11) Publication number: 0 349 296 A2

# (12) EUROPEAN PATENT APPLICATION

(21) Application number: 89306562.3

(22) Date of filing: 28.06.89

(51) Int. Cl.5: C 07 D 501/06, C 07 D 501/20

(30) Priority: 28.06.88 JP 158022/88

(43) Date of publication of application: 03.01.90 Bulletin 90/01

(84) Designated Contracting States: DE FR GB IT

(71) Applicant: AJINOMOTO CO., INC.
5-8, Kyobashi 1-chome, Chuo-ku
Tokyo 104 (JP)

MOCHIDA PHARMACEUTICAL CO., LTD.
7, Yotsuya 1-chome
Shinjuku-ku Tokyo 160 (JP)

(72) Inventor: Watanabe, Kazuhiro c/o Central Research Lab.
Ajinomoto Co. Inc. No. 1-1 Suzuki-cho Kawasaki-ku
Kawasaki-shi Kanagawa-ken (JP)

Kakizaki, Fusayoshi c/o Central Research Lab.
Ajinomoto Co. Inc. No. 1-1 Suzuki-cho Kawasaki-ku
Kawasaki-shi Kanagawa-ken (JP)

Arai, Isao c/o Central Research Lab.
Ajinomoto Co. Inc. No. 1-1 Suzuki-cho Kawasaki-ku
Kawasaki-shi Kanagawa-ken (JP)

Murakami, Kimihiro
No. 977-4, Kawashimata
Gotenba-shi Shizuoka-ken (JP)

Kato, Kazuo
No. 29-6, Fujimidai
Mishima-shi Shizuoka-ken (JP)

(74) Representative: Harrison, David Christopher et al
MEWBURN ELLIS 2 Cursitor Street
London EC4A 1BQ (GB)

The title of the invention has been amended (Guidelines for Examination in the EPO, A-III, 7.3).

(54) **Process for the acylation of 7-aminocephalosporanic acid or its derivatives.**

(57) An amide compound is made by the dehydrative condensation of a carboxylic acid with a 7-amino cephalosphoranic acid, in the presence of acid. In one process the amide compound is a 7-substituted amino-3-substituted methylcephem carboxylic acid.

EP 0 349 296 A2

**Description**

## PROCESS FOR PREPARING AMIDE COMPOUNDS

The present invention relates to a novel process for preparing amide compounds, which are utilizable for production of 7-substituted amino-3-substituted methylcephem-carboxylic acids useful as drugs such as antibiotics, or intermediates thereof.

It is known from JP-A-142987/1985 that amide compounds can be prepared by reacting 7-aminocephalosporanic acids and carboxylic acids in the presence of a dehydration condensing agent for example, dicyclohexylcarbodiimide. However, the yield of this process is poor.

It is therefore desired to improve the process described above and develop one which can produce highly pure amide compounds in a simple manner in a high yield, and which should threfore be of industrial importance.

As a result of extensive investigations to solve the foregoing problem, the present inventors have found that the object can be achieved by reacting 7-aminocephalosporanic acid or derivative thereof with a carboxylic acid in the presence of a dehydration condensing agent and acid or acid complex.

As the dehydration condensing agent, any compound conventionally used for amidation may be used; for example, carbodiimides of the formulas $R^1 - N = C = N - R^2$ may be used, wherein $R^1$ and $R^2$, which may be the same or different, each represents an alkyl group having 1 to 5 carbon atoms, an aryl group having 6 to 10 carbon atoms or an aralkyl group having 7 to 20 carbon atoms, a cycloalkyl group having 6 to 10 carbon atoms, any of which may optionally be substituted.

The acid or the acid complex may be a sulfonic acid represented by formula: $R^3SO_3H$, wherein $R^3$ represents a hydroxy group, an alkyl group having 1 to 5 carbon atoms, an aryl group having 6 to 10 carbon atoms, a cycloalkyl group having 6 to 10 carbon atoms or a aralkyl group having 7 to 20 carbon atoms, which may optionally be substituted, a halohydrogenic acid HX wherein X represents a halogen atom, a Lewis acid or a Lewis acid complex.

Example of the Lewis acid inolude halogen compounds of aluminium, tin, zinc, boron or titanium. Examples of the Lewis acid complex include dialkyl ether complexes with diethyl ether, di-n-propyl ether, di-n-butyl ether, etc.; fatty acid complexes with acetic acid, propionic acid, etc.; nitrile complexes with acetonitrile, propionitrile, etc.; carboxylic acid ester complexes with ethyl acetate, etc.; phenol complexes with phenols, etc. When a reaction solvent is used, this may be, for example, a halogen-containing organic solvent such as dichloromethane, chloroform, carbon tetrachloride, 1.2-dichloroethane, 1,1,1-trichloroethane, etc.; an alcoholic organic solvent such as methanol, ethanol, isopropyl alcohol, etc.; a ketonic organic solvent such as acetone, methyl ethyl ketone, acetophenone, etc.; an etheric organic solvent such as tetrahydrofuran, diethyl ether, dioxan, etc., or an admixture of two or more such solvents.

Utilizing the present invention, 7-substituted amino-3-substituted methylcephem-carboxylic acids can also be prepared.

That is, a 7-aminocephalosporanic acid derivative represented by the general formula I:

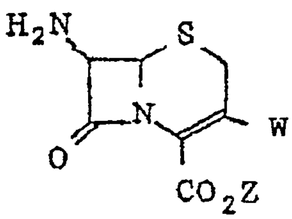

I

wherein W represents a hydrogen, ethenyl, 2-carboxyethenyl, chlorine, methoxy or a functional group $-CH_2Y$, wherein Y represents a hydrogen atom or a nucleophilic compound residue, and Z represents a hydrogen atom, a metal atom, or an alkyl group having 1 to 5 carbon atoms, an aryl group having 6 to 10 carbon atoms, a cycloalkyl group having 6 to 10 carbon or a aralkyl group having 7 to 20 carbon atoms, any of which may optionally be substituted, is reacted with a compound represented by general formula IV:

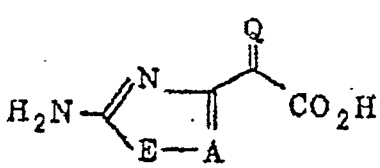

IV

wherein A represents C-H or N; E represents S or O; Q represents =O or a functional group shown by $=N R^4$ (wherein $R^4$ represents hydroxy group or an alkoxyl groupwhich may optionally be substituted; and which may be in syn- or anti-form); in an organic solvent in the presence of a dehydration condensing agent represented by general formula II:

$R^1 - N = C = N - R^2$ II

wherein $R^1$ and $R^2$, which may be the same or different, each represents an alkyl group having 1 to 5 carbon

atoms, an aryl group having 6 to 10 carbon atoms, a cycloalkyl group having 6 to 10 carbon atoms or a aralkyl group having 7 to 20 carbon atoms, which may optionally be substituted, respectively, and an acid, for example, a sulfonic acid represented by general formula III:

$R^3SO_3H$ III

wherein $R^3$ represents a hydroxy group, an alkyl group having 1 to 5 carbon atoms, an aryl group having 6 to 10 carbon atoms, a cycloalkyl group having 6 to 10 carbon atoms, or a aralkyl group having 7 to 20 carbon atoms, which may optionally be substituted; or a hydrohalogenic acid shown by HX, wherein X represents a halogen atom, or, a Lewis acid or Lewis acid complex; to give compounds represented by general formula VI:

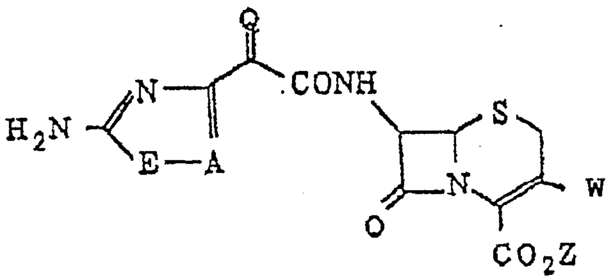
VI

wherein symbols have the same significances described above, in a high yield and high purity by industrially simple procedures.

Very many methods are known for reacting the amino group at the 7-position of 7-aminocephalosporanic acids with carboxylic acids or salts or acid halides thereof for change at the 7-position.

However, amino group-containing compounds as shown in formula IV give the product in a low yield, through conventional condensation.

Examples of the nucleophilic compound residue Y in formulae I and VI include hydroxy, mercapto, carbamoyl, cyano, azido, amino, carbamoyloxy, carbamoylthio or thiocarbamoyloxy which may optionally be substituted with an alkyl (methyl, ethyl, propyl, etc.), an acyloxy (acetyloxy, propionyloxy, butyryloxy, benzoyloxy, p-chlorobenzoyloxy, p-methylbenzoyloxy, etc.); a quaternary ammonium group; or hydroxyphenyl, sulfamoyloxy, an alkylsulfonyloxy, (cis-1,2-epoxypropyl) phosphono, etc. Y also represents a hetero ring bound via S. Herein, "hetero ring" refers to a 5- to 6-membered ring or a 7- to 14-membered fused ring containing (or each containing) 1 to 5 hetero atoms selected from O, S and N. Examples of the hetero ring which can be often used include pyridyl, N-oxidopyridyl, pyrimidyl, pyridazinyl, N-oxidopyridazinyl, pyrazolyl, diazolyl, thiazolyl, 1,2,3-thiadiazolyl, 1,2,4-thiadiazolyl, 1,3,4-thiadiazolyl, 1,2,5-thiadiazolyl, 1,2,3-oxadiazolyl, 1,2,4-oxadiazolyl, 1,3,4-oxadiazolyl, 1,2 5-oxadiazolyl, 1,2,3-triazolyl, 1,2,4-triazolyl, 1H-tetrazolyl, 2H-tetrazolyl, S-triazolo(1,5-a)pyrimidine, tetrazolo(1,5-b)pyridazinyl, pyrazolo(1,5-a)pyrimidinyl, isoindolyl,1,2,4-triazinyl,1,3,5-triazinyl etc. These hetero rings may also have substituents, for example, a lower alkyl group such as methyl, ethyl, propyl, etc. or as a cycloalkane fused to the hetero ring, a lower alkoxy group such as methoxy, ethoxy, etc.; a halogen such as chlorine, bromine, etc.; a halogeno-substituted alkyl such as trifluoromethyl, trichloromethyl, etc.; hydroxy or hydroxymethyl group, mercapto group, amino group, carboxyl or carboxymethyl group, an ester group, carbamoyl or carbamoylmethyl group, etc. Examples of the quaternary ammonium group which can be often used include phridinium, 3-methylpyridinium, 4-methylpyridinium, 3-chloropyridinium, 3-bromopyridinium, 3-iodopyridinium, 4-carbamoylpyridinium, 4-(N-carbomethoxycarbamoyl)pyridinium, 4-(N-cyanocarbamoyl) pyridinium, 4-(hydroxymethyl)pyridinium, 4-(carboxylmethyl) pyridinium, 4-(trifluoromethyl)pyridinium, quinolinium, picolinium, lutidinium, etc.

The $CO_2Z$ group in formulae I and VI means carboxylic acid or a salt with alkali metal or alkaline earth metal such as sodium, potassium, calcium, etc.; or an ester with e.g. an alkyl, aryl or aralkyl group such as methyl, ethyl, propyl, vinyl, phenyl, benzyl, diphenylmethyl, triphenylmethyl, etc. These esters may have any substituent thereon so long as the substituent does not inhibit the reaction. In formula II, the alkyl, aryl or aralkyl group in formula II which may optionally be substituted may be any substituent as far as it does not inhibit the reaction in the present invention.

In formula III, $R^3$ means a hydroxy, alkyl, aryl or aralkyl group exemplified by methyl, ethyl, propyl, vinyl, camphanyl, phenyl, p-methylphenyl, etc. These groups may be substituted with any substituent as far as it does not inhibit the reaction.

Examples of the Lewis acid which can be used are halides of aluminum, tin, zinc, boron or titanium. As the complex of Lewis acid, mention may be made of a dialkyl ether complex with diethyl ether, di-n-propyl ether, di-n-butyl ether, etc.; a fatty acid complex with acetic acid, propionic acid, etc.; a nitrile nitrile complexes with acetonitrile, propionitrile, etc.; carboxylic acid ester complexes with ethyl acetate, etc.; phenol complexes with phenols, etc. The alkoxy group in $R^4$ of formula V may have any substituent thereon so long as the substituent does not inhibit the reaction.

In a preferred embodiment, the process of the present invention can involve reacting the compound of formula I with the carboxylic acid of formula IV in an organic solvent in the presence of the dehydration condensing agent represented by formula II and the acid.

The solvent used for the reaction may be any organic solvent inasmuch as it does not adversely affect the reaction. Preferred examples are a halogen-containing organic solvent such as dichloromethane,, chloroform,

carbon tetrachloride, 1,2-dichloroethane, 1,1,1-trichloroethane, etc.; an alcoholic organic solvent such as methanol, ethanol, isopropyl alcohol, etc.; a ketonic organic solvent such as acetone, methyl ethyl ketone, acetophenone, etc.; an etheric organic solvent such as tetrahydrofuran, diethyl ether, dioxan, etc.; an esteric organic solvent such as ethyl acetate, etc. These solvents may also be used as admixture of two or more.

The amount of the acid of formula III may be at least 0.1 equmolar amount based on the compound of formula I. In general, the reaction rate varies greatly depending upon kind of the solvent and carboxylic acid of formula IV. Therefore, it is desired that amounts of the dehydration condensing agent shown by formula II and the acid of formula III be appropriately increased or reduced depending upon respective cases.

The reaction temperature is not particularly critical but is generally in a range of -20 to 10 °C and the reaction time is generally several minutes to several hours.

However, the reaction conditions described above are not limited thereto and may be appropriately chosen depending upon reactants and solvents to achieve the object.

Examples

Next, the present invention will be described by referring to the examples below.

Example 1

Production of diphenylmethyl (6R,7R)-7-[2-(2-amino-4-thiazolyl)-2-[Z-[diphenylmethyloxycarbonyl(3,4-dihydroxyphenyl)methyl[oxy imino]acetamido]-3 [[2-diphenylmethyloxycarbonyl-5-methyl-s-triazolo(1,5-a)pyrimidin-7-yl]thiomethyl]-8-oxo-5-thia-1-azabicyclo[4.2.0]oct-2-ene-2-carboxylate:

In 25 ml of 1,2-dichloroethane was dissolved 2.78 g of diphenylmethyl (6R,7R)-7-amino-3-[[2-diphenylmethyloxycarbonyl-5-methyl-s-triazolo(1,5-a)pyrimidin-7-71]thiomothyl]-8-oxo-5-thia-1-azabicyclo[4.2.0]oct-2-ene-2-carboxylate. In 13 ml of methanol was dissolved 2.85 g of 2-(2-amino-4-thiazolyl)-2-[z-[diphenyl-methyloxycarbonyl(3,4-dihydroxyphenyl)methyl]ox-iminoacetic acid and the solution was added to the 1,2-dichloroethane solution. To the mixture was added 0.3 ml of methanesulfonic acid followed by stirring. The mixture was then cooled to 5 °C. In 2 ml of 1,2-dichloroethane was dissolved 1.5 g of dicyclohexylcarbodiimide and the solution was dropwise added to the reaction solution. Forty minutes later, insolubles were filtered off. After the filtrate was concentrated, ethyl acetate was added to the residue and insolubles were filtered off. After washing with saturated sodium chloride aqueous solution and drying over anhydrous sodium sulfate, the filtrate was concentrated and the residue was purified by silica gel column chromatography to give 6.4 g of the title compound.

IR spectrum (KRr $cm^{-1}$)

1780, 1742, 1737, 1507, 1249, 1205, 1182

NMR spectrum ($CDCl_3$ ppm)

7.5-7.2 (35H, m), 7.0 (1H, s), 6.82 (1H, s), 6.76 (1H, s), 5.9 (1H, s) 5.8 (1H, dd), 4.9 (1H, d) 4.2 (2H, bs), 3.7 (2H, ABq), 2.6 (3H, s)

Example 2

Production of diphenylmethyl (6R,7R)-7-(2-amino-4-thiazolyl)-2-[Z-[diphenylmethyloxycarbonyl(3,4-dimethyl methylenedioxy phenyl)methyl]oxyimino]acetamido]-3-[[2-diphenylmethyloxycarbonyl-5-methyl-s-triazolo(1,5-a)pyrimidin-7-yl]thiomethyl]-8-oxo-5-thia-1-azabicyclo[4.2.0]oct-2-ene-2-carboxylate:

In 30 ml of 1,2-dichloroethane was dissolved 2.78 g of diphenylmethyl (6R,7R) 7 amino-3-[[2-diphenylmethyloxycarbonyl-5-methyl-s-triazolo(1,5-a)pyrimidin-7-yl]thiomethyl]-8-oxo-5-thia-1-azabicyclo[4.2.0]oct-2-ene-2-carboxylate and, 2.90 g of 2-(2-amino-4-thiazolyl)-2-[Z-[diphenylmethyloxycarbonyl(3,4-dimethyl methyl enedioxyphenyl)methyl]oxyiminoacetic acid was added to the solution and further 0.3 ml of methanesulfonic acid was added to the mixture. While stirring, the mixture was cooled to 5 °C. In 2 ml of 1,2-dichloroethane was dissolved 1.5 g of dicyclohexylcarbodiimide and the solution was dropwise added to the reaction solution. Twenty minutes later, insolubles were filtered off. After the filtrate was concentrated, ethyl acetate was added t the residue and sinsolubles were filtered off. After washing with saturated sodium chloride acqueous solution and drying over anhydrous sodium sulfate, the filtrate was concentrated and the residue was purified by silica gel column chromatography to give 6.3 g of the title compound.

NMR spectrum ($CDCl_3$ ppm)

7.5-7.1 (35H, m), 7.0 (1H, s), 6.9 (1H, s), 6.8 (1H, s), 5.9 (1H, s), 5.8 (1H, dd) 5.2 (1H, d), 3.5 (2H, ABq), 2.5 (3H, s), 1.6 (6H, s)

Example 3

Production of diphenylmethyl (6R,7R)-7-[2-(2-amino-4-thiazolyl)-2-[Z-methoxyimino]acetamido]-3-acetoxymethyl-8-oxo-5-thia-1-aza-bicyclo[4.2.0]oct-2-ene-2-carboxylate:

To 25 ml of 1,2-dichloroethane was added 1.83 g of diphenylmethyl (6R,7R)-7-amino-3-acetoxymethyl-8-oxo-5-aza-bicyleo[4.2.0]oct-2-ene-2 carboxylate. To 25 ml of tetrahydrofuran was added 1.0 g of 2-(2-amino-4-thiazolyl)-2-[z-methoxyimino]acetic acid and the mixture was added to the 1,2-dichloroethane solution. To the mixture was added 0.3 ml of methanesulfonic acid to dissolve. Furthermore, a solution of 1.5 g of dicyclohexylcarbodiimide in 2 ml of 1,2-dichloroethane was added to the solution. The mixture was then cooled to -5 °C and stirred for an hour. After insoluble matters were filtered off and the filtrate was concentrated, ethyl acetate was added to the residue followed by filtration. After washing with saturated sodium chloride acqueous solution and drying over anhydrous sodium sulfate, the filtrate was concentrated. The residue was purified by silica gel column chromatography to give 2.91 g of the title compound.

NMR spectrum ($CDCl_3$ ppm)

7.5-7.1 (11H, m), 6.8 (1H, s), 5.8 (1H, dd), 5.1 (1H, d), 4.6 (2H, bs), 4.1 (3H, s), 3.7 (2H, ABq), 2.1 (3H, s)

Examples 4 through 7

The procedures of Example 1 were repeated except using:

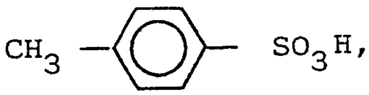, HCL, $BF_3OEt_2$, $H_2SO_4$

HCL, $BF_3OEt_2$, $H_2SO_4$

as the acid, respectively. The results are shown in the following table. For purpose of comparison, the results obtained with a case where no acid was added are also shown in the table.

Examples 4 through 7

| Compound [IV] | Compound [I] | Acid Added | Yield [%] |
|---|---|---|---|

| | | | | |
|---|---|---|---|---|
| Ex. 4 | 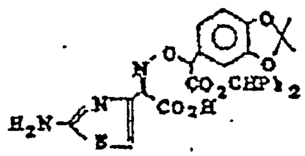 | 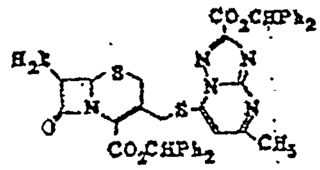 | 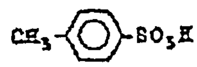 | 97 |
| Ex. 5 | 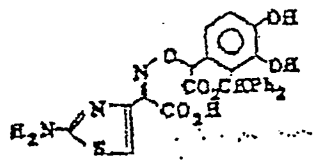 | ditto | HCl | 98 |
| Ex.6 | ditto | ditto | $BF_3OEt_2$ | 90 |
| Ex. 7 | ditto | ditto | $H_2SO_4$ | 77 |
| Comparison | ditto | ditto | - | 70 |

Condensing agent:

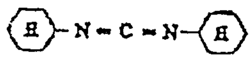

Solvent: 1,2-dichloroethane

As is clear from the foregoing, according to the present invention, the desired amide compounds can be produced from 7-aminocephalosporanic acid or derivatives thereof in a high yield and high purity by industrially simple procedures.

**Claims**

(1) A process for preparing an amide compound which comprises reacting a 7-aminocephalosporanic acid or derivative thereof with a carboxylic acid in the presence of a dehydration condensing agent characterised in that an acid or an acid complex is present.

(2) A process according to Claim 1, wherein said dehydration condensing agent is a carbodiimide represented by formula:

$R^1 - N = C = N - R^2$

wherein $R^1$ and $R^2$, which may be the same or different, each represents an alkyl group having 1 to 5 carbon atoms, an aryl group having 6 to 10 carbon atoms or a cycloalkyl group having 6 to 10 carbon atoms, an aralkyl group having 7 to 20 carbonations, any of which may optionally be substituted.

(3) A process according to Claim 1 or Claim 2, wherein said acid or acid complex is selected from a protonic acid, a Lewis acid and a Lewis acid complex.

(4) A process according to Claim 1 or Claim 2, wherein said acid is a sulfonic acid represented by formula: $R^3SO_2H$, wherein $R^3$ represents a hydroxy group, an alkyl group having 1 to 5 carbon atoms, an aryl group having 6 to 10 carbon atoms, a cycloalkyl group having 6 to 10 carbon atoms or an aralkyl grup having 7 to 20 carbon atoms, any of which may optionally be substituted; or an acid HX wherein X represnts a halogen atom.

(5) A process according to any one of preceding claims, wherein the reaction is carried out in a halogen-containing organic solvent, an alcoholic organic solvent, a ketonic organic solvent, an etheric organic solvent, an esteric organic solvent, or a mixture of any of these.

(6) A process according to any one of preceding claims, wherein the 7-aminocephalosporanic acid or derivative(s) thereof is represented by the general formula:

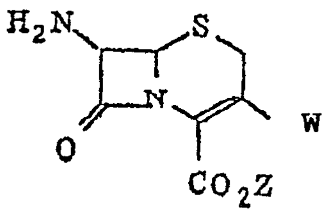

the carboxylic acid is a compound represented by the general formula:

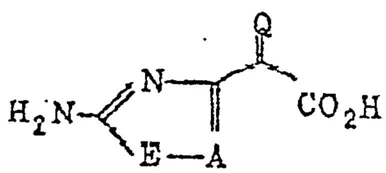

and the amide compound is a 7-substituted amino-3-substituted methylcephem-carboxylic acid;

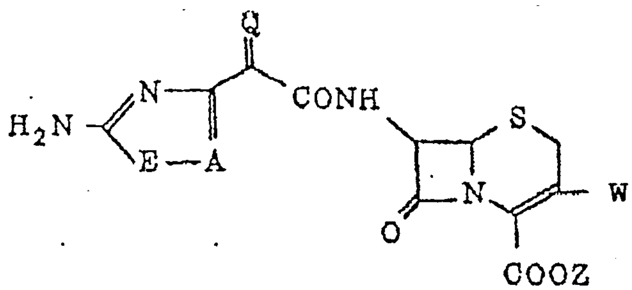

wherein W represents a hydrogen, ethenyl, 2-carboxyethenyl, chlorine, methoxy or a functional group $-CH_2Y$ wherein Y represents a hydrogen atom or a nueleophilic compound residue, Z represents a hydrogen atom, a metal atom, or an alkyl group having 1 to 5 carbon atoms, an aryl group having 6 to 10 carbon atoms, a cycloalkyl group having 6 to 10 carbon atoms or an aralkyl group having 7 to 20 carbon atoms any of which may optionally be substituted, and A represents >C-H or >N; >E represents >S or >O; Q represents =O or a functional group -N-$R^4$, (wherein $R^4$ represents hydroxy group or an alkoxyl group which may optionally be substituted and which group -N-$R^4$ may be in the syn- or anti-form).